# EUROPEAN PATENT APPLICATION

(11) **EP 3 514 540 A1**
(43) Date of publication of application: **24.07.2019**
(21) Application number: 17862802.0
(22) Date of filing: 18.10.2017
(51) Int. Cl.: G01N 33/543

(54) **BIOSENSOR**

(30) Priority: 20.10.2016 KR 20160136342
(71) Applicant: Plexense, Inc., Gyeonggi-do 17015 (KR)
(72) Inventor: HWANG, Hyejin, Yongin-si Gyeonggi-do 16861 (KR); JEON, Jinwoo, Seoul 06764 (KR); LEE, Jiyoung, Gyeryong-si Chungcheongnam-do 32837 (KR)
(74) Representative: Grosse, Felix Christopher
(86) International application number: PCT/KR2017/011520
(87) International publication number: WO 2018/074832

(57) **Abstract**

A biosensor is disclosed. The biosensor according to the embodiments of the present invention includes at least one sensor strip including a sensor body with a predetermined length, a plurality of reaction chambers recessed from one surface of the sensor body, and one or more detection structures arranged across the internal space of each reaction chamber and a fixing plate having a surface to which the sensor strip is detachably attached.

## Description

### TECHNICAL FIELD

The present invention relates to a biosensor.

### BACKGROUND ART

The metal nanostructure has an electric dipole characteristic caused by the collective oscillation of electrons in the nanostructure conduction band. Thus, the nanostructure strongly absorbs or scatters the light of a specific frequency incident from the outside. This phenomenon is called Localized Surface Plasmon Resonance (LSPR). Herein, the absorbance characteristic of the metal nanostructure with respect to light is dependent on the metal nanostructure and highly sensitive to the complex permittivity (complex refractive index) of the medium around the surface of the metal nanostructure. Therefore, LSPR can be utilized as a sample analysis method for biomolecules and chemical species.

Analytical methods utilizing such an LSPR phenomenon for assaying biological or non-biological samples have been investigated to overcome disadvantages, such as complex sample processing and long analysis time, of existing fluorescence-based analysis methods.

Common methods for analyzing biological samples such as nucleic acids and proteins can be divided into two major areas as follows. The first one is a method of analyzing the concentration of a sample by measuring optical absorbance using an ultraviolet-visible (UV-VIS) spectroscopic method. In the method, an absorbance is measured by passing light of a certain intensity through a sample and then comparing intensity of light before and after the passage. Such an optical absorbance measurement method measures only the concentration of a specific functional group contained in the sample. Therefore, there exists inconvenience of applying an additional analytical method or more in order to quantitatively analyze the reactivity and activity of a specific binding substance in a biological reaction. Furthermore, the method offers a low analytical sensitivity of 10-6M and thus it is not suitable for analyzing a biological sample that typically requires a high analytical sensitivity of 10-12M.

The second one is to utilize enzyme immunoassay, as disclosed in the prior art patent document KR 2013 - 0 014 713. Enzyme immunoassay is a method commonly used for quantitatively analyzing the reactivity and activity of a specific sample at a high analytical sensitivity of 10-12M. The enzyme immunoassay uses a quantitative analysis method in which a sample is analyzed using an enzyme-labeled antibody formed by chemical binding of an enzyme such as peroxidase or galactosidase with an antibody in a target-specific antigen-antibody reaction. Alternatively, fluorescence immunoassay can be used in which a sample is analyzed using an antigen or antibody labeled with a fluorescent dye such as fluorescein and rhodamine and a fluorescence analyzer.

These analytical methods are widely used because they permit to analyze, with an excellent detection sensitivity, the reactivity and activity of the reaction between a reactant and a target analyte in a sample. However, they still have problems of a long assay time and high assay cost because of complicated sample processing, labeling of a sample or target analyte with a fluorescent dye, or use of an expensive analyzer. In particular, enzyme immunoassay or fluorescence immunoassay has difficulties in rapid screening of a large number of libraries during drug development or biomarker development due to a long assay time and necessity of using a separate target-specific antibody depending on the target analyte.

Therefore, there is a desperate need for a solution to the problem of the conventional sample analysis method.

### DETAILED DESCRIPTION OF THE INVENTION

The present invention is intended to solve the aforementioned problems of the conventional arts. One aspect of the present invention is, in a plurality of reaction chambers recessed from one surface of a sensor strip, to arrange detection structures that react with a sample and induce an LSPR phenomenon. That provides a biosensor which can easily induce a sample reaction without separate sample pretreatment process.

### MEANS FOR SOLVING THE PROBLEMS

The biosensor of the present invention includes at least one sensor strip including a sensor body with a predetermined length, a plurality of reaction chambers recessed from one surface of the sensor body, and one or more detection structures arranged across the internal space of each reaction chamber.
The biosensor of the present invention further includes a fixing plate having a surface to which the sensor strip is detachably attached.

In the biosensor of the present invention, the detection structure includes a substrate arranged across the internal space of the reaction chamber; and a thin film layer that is formed on at least one of the both surfaces of the substrate by dispersedly disposing conductive nanoparticles or nanostructures that cause the LSPR phenomenon and react with a target analyte in a sample.

In the biosensor of the present invention, the detection structure is provided in plurality and the detection structures are horizontally spaced apart from each other in the reaction chamber.

The biosensor of the present invention further includes sample injection holes recessed from one surface of the sensor body so as to be in communication with the reaction chambers.

In the biosensor of the present invention, the fixing plate includes light passing holes perforated along its thickness direction so that the light irradiated from the direction of the surface of the fixing plate enters the detection structure or the light irradiated to the direction of the surface of the fixing plate passes through it.

In the biosensor of the present invention, the aforementioned light passing hole is formed in plurality to correspond one-to-one to the detection structures arranged across the internal space of each reaction chamber.

The biosensor of the present invention further includes an insertion protrusion protruding from one surface of the fixing plate wherein the sensor body is recessed or perforated to form an insertion recess into which the insertion protrusion is inserted such that the sensor strip is attached to the fixing plate.

The biosensor of the present invention further includes a fixing protrusion spaced from the insertion protrusion and protruding from one surface of the fixing plate such that the insertion protrusion comes into contact with an inwardly recessed corner of one end of the sensor body when inserted into the insertion hole.

The features and advantages according to the present invention will become apparent from the following description with reference to the accompanying drawings.

Prior to the detailed description of the invention, it should be understood that the terms and words used in the specification and the claims are not to be construed as having common and dictionary meanings but are construed as having meanings and concepts corresponding to the technical spirit according to the disclosed embodiments in view of the principle that the inventor can define properly the concept of the terms and words in order to describe his/her invention with the best method.

### EFFECTS OF THE INVENTION

A biosensor according to the present invention quantitatively detects a sample by inducing an LSPR phenomenon on the thin film layer of metal nanoparticles or nanostructures dispersedly disposed on at least one of one surface and the other surface of the substrate arranged across the internal space of each reaction chamber of the sensor strip. The biosensor can easily induce the reaction between biological samples or between biological and non-biological samples, without a separate sample pretreatment process.

In the biosensor according to the present invention, the sensor strip includes a plurality of the reaction chambers. A plurality of the detection structures configured to bind specifically with target analytes are arranged to be stacked in parallel across the internal space of each reaction chamber. In this regard, various kinds of protein quantitative analysis or immunoassay can be performed at the same time, thus reducing the time required for sample analysis.

In addition, a sample analysis method using a biosensor of the present invention is based on an LSPR phenomenon and thus does not necessitate chromophore labeling, unlike enzyme immunoassay that requires a complicated step of labeling a sample molecule with a chromophore. Therefore, the biosensor permits to quantitatively analyze a sample through a simple detection process only with a visible light spectroscopic analyzer.

### BRIEF DESCRIPTION OF THE DRAWINGS

- Fig. 1: is a perspective view of a biosensor according to an embodiment of the present invention.
- Fig. 2: is a magnified view of the detection structure shown in Fig. 1.
- Fig. 3: is a perspective view of a sensor strip according to another embodiment of the present invention.
- Fig. 4: is a perspective view of a biosensor according to another embodiment of the present invention.

### BEST MODE FOR CARRYING OUT THE INVENTION

The objectives, specific advantages, and novel features of the present invention will become more apparent from the following detailed description and preferred embodiments with reference to the appended drawings. It should be noted that the same reference numerals are denoted to the elements of the drawings in the present specification with the same numerals as possible, even if they are displayed in other drawings. Also, the terms "the first", "the second" and the like are used to distinguish one element from another and thus the element is not limited thereto. Hereinafter, in the description of the present invention, a detailed explanation of related known arts which may unnecessarily obscure the gist of the present invention will be omitted.

Hereinafter, preferred embodiments of the present invention will be described in detail with reference to the appended drawings.

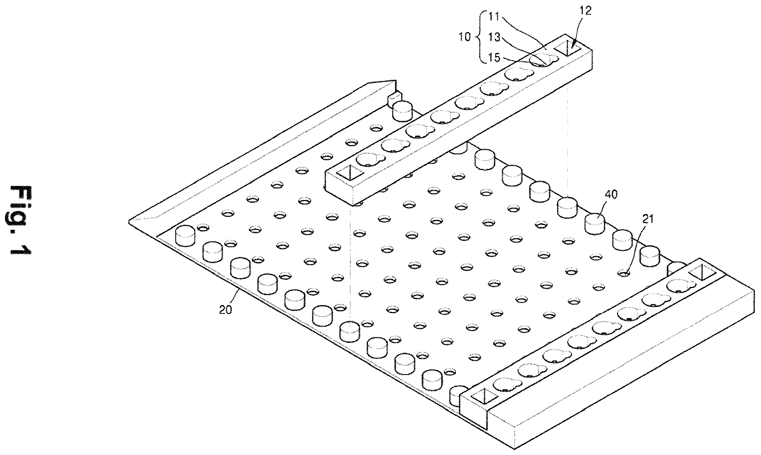

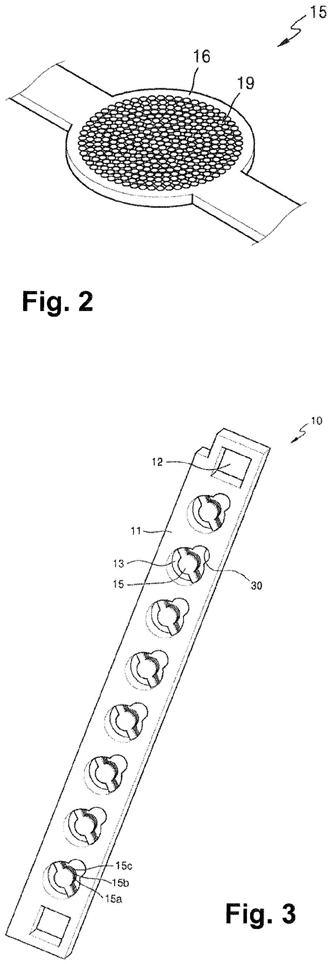

Fig. 1 is a perspective view of a biosensor according to an embodiment of the present invention and Fig. 2 is a magnified view of the detection structure shown in Fig. 1.

As illustrated in Fig. 1 and Fig. 2, the biosensor according to an embodiment of the present invention includes at least one sensor strip (10) including a sensor body (11) with a predetermined length, a plurality of reaction chambers(13) recessed from one surface of the sensor body(11), and one or more detection structures(15) arranged across the internal space of each reaction chamber(13).

Surface plasmon resonance (SPR) refers to a phenomenon of the propagation of surface plasmon polaritons (SPPs) which are generated on or near the surface of conductive materials by coupling of electrons and photons having a specific wavelength. In general, SPR is a phenomenon of the collective oscillation of conduction band electrons propagating along the interface between a metal with a negative dielectric constant and a medium with a positive dielectric constant. SPR results in enhanced intensity in comparison with an incident electromagnetic wave and shows characteristics of an evanescent-wave which exponentially decays as getting far-off perpendicularly from the interface.

SPR can be classified as a propagating surface plasmon resonance (PSPR) observed at the interface between a dielectric material and a 10-200 nm-thick flat metal surface; and localized surface plasmon resonance (LSPR) observed from nanoparticles or nanostructures. A biosensor based on LSPR detects a change in the LSPR wavelength showing a maximum absorption or scattering which depends on a change of the chemical and physical environment on the surface (for example, a change in refractive index of a medium near the surface) of the nanoparticles or nanostructures. The detection of the LSPR wavelength change permits to distinguish specific molecules or to analyze concentration of specific molecules in a medium; LSPR is highly sensitive to the change of refractive index and that allows label-free detection. A biosensor according to the present invention is fabricated such that LSPR is applied.

Specifically, the biosensor according to an embodiment of the present invention includes sensor strips (10), each of them includes a sensor body (11), reaction chambers (13), and detection structures (15). Each sensor strip (10) has a structure in which reaction chambers (13) are formed in the sensor body (11) in the shape of a plate with a predetermined length and width and the detection structures (15) are arranged across the internal space of each reaction chamber (13).

The reaction chambers (13) are recessed from one of the outer surfaces of the sensor body (11) and a plurality of the reaction chambers (13) are arranged along the lengthwise direction of the sensor body (11). A sample solution is injected inside the reaction chamber (13) and a target analyte in the sample solution is detected by the detection structures (15) arranged therein.

The detection structures (15) are arranged across the internal space of each of the plurality of reaction chambers (13) such that they are immersed in the sample solution accommodated in the reaction chamber (13). Accordingly, the detection structures (15) react with a target analyte in the sample solution and analyze the sample solution by generating LSPR by the light irradiated outside.

Here, the detection structure (15) may include a substrate (16) and a thin film layer (19) (referring to Fig. 2). The substrate (16) is a component arranged across the internal space of the reaction chamber (13). The substrate (16) may be optically transparent or opaque substrate (16), but the optically transparent substrate (16) is preferable. The optically transparent substrate (16) may be made of, for example, glass or a polymer material having a certain degree of optical transparency. The polymer material may comprise polycarbonate (PC), polyethylene terephthalate (PET), polymethyl methacrylate (PMMA), triacetyl cellulose (TAC), cyclic olefin, polyarylate, polyacrylate, polyethylene naphthalate, polybutylene terephthalate or polyamide. However, the polymer material is not necessarily limited thereto. The optically opaque substrate (16) may be made of sapphire, silicon single crystal. However, the material of the substrate (11) is not limited to the aforementioned materials and various other materials can be utilized in consideration of the conditions of the target analyte, the fabrication process, and the like. The thin film layer (19) is a layer formed on at least one of the both surfaces of the substrate (16).

The thin film layer (19) is a layer formed on at least one of the both surfaces of the substrate (16) and is formed by dispersedly disposing conductive nanoparticles or nanostructures that cause LSPR. The thin film layer (19) may be formed on only one surface of the substrate (16) or on both surfaces of the substrate (16). At this time, the conductive nanoparticles or nanostructures may have any shape selected from a nanosphere, a nanotube, a nanocolumn, a nanorod, a nanopore, a nanowire, or combinations thereof. The nanoparticles or nanostructures may be completely filled, porous or hollowed depending on the shape. The conductive nanoparticles or nanostructures may be conductive particles of carbon, graphite, metalloid, metal, metalloid alloy, metal alloy, conductive metal oxide, conductive metal nitride; or core-shell structure particles in which a conductive layer such as a metal thin film is coated on an insulating core. However, the conductive nanoparticles or nanostructures are not necessarily limited to the aforementioned shapes and materials.

On the other hand, the conductive nanoparticles or nanostructures are immobilized on the substrate (16) by a binder wherein the binder may be an ionic polymer such as poly diallyldimethylammonium chloride, poly allylamine hydrochloride, poly 4-vinylbenzyltrimethyl ammonium chloride, polyethyleneimine, poly acrylic acid, poly sodium 4-styrene sulfonate, poly vinylsulfonic acid, poly sodium salt, poly amino acids or a mixture thereof. However, the binder is not limited to the aforementioned polymer, as long as it is a material capable of immobilizing nanoparticles or nanostructures on the substrate (16).

The detection structure (15) is arranged across the internal space of the reaction chamber (13). Accordingly, the thin film layer (19) of the detection structure (15) reacts with a target analyte in a sample solution when the sample solution is injected in the reaction chamber (13). At this time, a detection substance that specifically binds with the target analyte in the sample solution may be immobilized on the thin film layer (19) in order for the thin film layer (19) to bind with the target analyte. The detection substance may be, for example, a low molecular weight compound, an antigen, an antibody, a protein, a peptide, a DNA, an RNA, a PNA, an enzyme, an enzyme substrate, a hormone receptor, and a synthetic reagent having a functional group. However, the aforementioned detection substances are just exemplary ones and thus the detection substance is not necessarily limited thereto. The detection substance may comprise any known substances, including combinations of such substances, that combine with the target analyte. The detection substance is immobilized on the thin film layer (19), i.e., conductive nanoparticles or nanostructures, or on the binder; and specifically binds to the target analyte, thereby binding the target analyte to the thin film layer (13). However, the detection substance is not necessarily immobilized on the thin film layer (19).

The aforementioned detection structure (15) allows the sensor strip (10) to be used for protein quantitation, immunoassay and other assays. The biosensor may include one or more sensor strips (10) and each sensor strip (10) may include a plurality of the reaction chambers (13) and the detection structures (15). The presence of the plurality of reaction chambers (13) and the detection structures (15) in each of the sensor strips (10) enables simultaneous analysis of a plurality of samples. That is, different analyses can be done on the same sample; or different samples can be analyzed when different detection substances are immobilized on the thin film layers (19) of each reaction chamber (13). The opposite surface to the surface of the sensor strip (10) where the openings of the reaction chambers (13) are formed may be arranged on a fixing plate (20).

The fixing plate (20) has a shape of the plate with a predetermined width and thickness. The sensor strips (10) are detachably attached to one surface of the fixing plate (20). The sensor strips (10) can be attached to and detached from the fixing plate (20) by insertion protrusions (40) and insertion holes (12). The insertion protrusions (40) are inserted into and fixed to the insertion holes (12). The insertion holes (12) may be recessed or perforated so as to have a shape corresponding to the outer shape of the insertion protrusions (40). Due to their corresponding shapes, the insertion protrusions (40) are releasably withdrawn from the insertion holes (12). The insertion protrusions (40) may protrude from one surface of the fixing plate (20) and the insertion holes (12) may be formed on the opposite surface of the sensor body (11) of the sensor strip (10) so that the sensor strip (10) can be attached to and detached from the fixing plate (20). Alternatively, the insertion protrusions (40) may be formed on the sensor strip (10) and the insertion holes (12) may be formed in the fixing plate (20).

The detection structures (15) need to be irradiated with external light to cause LSPR. Accordingly, the fixing plate (20) may be perforated along its thickness direction to form light passing holes (21). The reaction chambers (13) are arranged on the light passing holes (21) formed in the fixing plate (20).

Here, a light causing LSPR can be irradiated onto one or the opposite surface of the fixing plate (20). The light irradiated onto one surface of the fixing plate (20) passes through the detection structure (15) and then passes through the fixing plate (20) through the light passing holes (21). On the other hand, the light irradiated onto the opposite surface of the fixing plate (20) passes through the fixing plate (20) through the light passing holes (21), and then enters into the detection structure (15) to cause LSPR. Meanwhile, in one sensor strip (10), a plurality of reaction chambers (13) are formed and a plurality of detection structures (15) are arranged in each reaction chamber (13). Thus, a plurality of light passing holes (21) are formed in the fixing plate (20) to correspond one-to-one to each set of detection structures (15).

In summary, a biosensor according to the present invention quantitatively detects a target analyte by inducing an LSPR phenomenon on the thin film layer (19) of metal nanoparticles or nanostructures dispersedly disposed on at least one of one surface and the other surface of the substrate (16) arranged across the internal space of the reaction chamber (13) of the sensor strip (10). The biosensor can easily induce the reaction between biological samples or between biological and non-biological samples, without a separate sample pretreatment process.

In addition, in the biosensor according to the present invention, the sensor strip (10) includes a plurality of the reaction chambers (13). A plurality of the detection structures (15) are arranged across the internal space of each reaction chamber (13). In this regard, various kinds of protein quantitative analysis or immunoassay can be performed at the same time, thus reducing the time required for sample analysis.

Furthermore, a sample analysis method is based on a LSPR phenomenon and thus does not necessitate chromophore labeling, unlike enzyme immunoassay that requires a complicated step of labeling a sample molecule with a chromophore. Therefore, the biosensor according to the present invention permits to quantitatively analyze a sample through a simple detection process only with a visible light spectroscopic analyzer.

### MODE FOR CARRYING OUT THE INVENTION

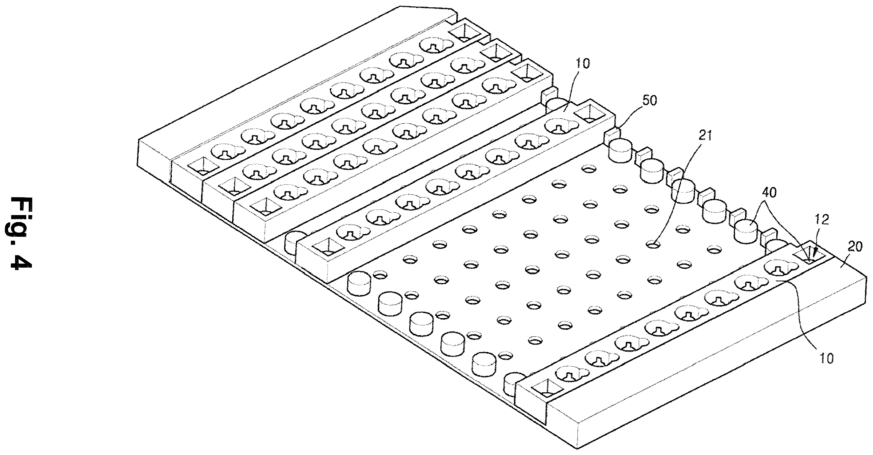

Fig. 3 is a perspective view of a sensor strip according to another embodiment of the present invention, and Fig. 4 is a perspective view of a biosensor according to another embodiment of the present invention.

As illustrated in Figs. 3 and 4, a sensor strip (10) according to an embodiment of the present invention may include a plurality of detection structures (15). Here, the plurality of detection structures (15a, 15b, 15c) may be spaced apart from each other along the depth direction of the reaction chamber (13). Thus, the substrate (16) is arranged to face the other substrate (16). The arrangement of the plurality of the substrates (16) can be parallel to each other but does not necessarily be parallel.

In addition, the biosensor according to an embodiment of the present invention may further include sample injection holes (30). Here, the sample injection holes (30) may be recessed from one surface of the sensor body (11) so as to be in communication with the inner space of the reaction chambers (13). The sample solution is injected into the reaction chambers (13) through the sample injection holes (30) and the detection structures (10) are immersed in the sample solution.

In addition, the biosensor according to an embodiment of the present invention may further include fixing protrusions (50) to more firmly fix sensor strips (10) to a fixing plate (20). The fixing protrusions (50) protrude from one surface of the fixing plate (20) and are arranged at predetermined intervals from insertion protrusions (40). The distances between the fixing protrusions (50) and the insertion protrusions (40) are determined such that each of the fixing protrusions (50) is brought into contact with the outer surface of one end of the sensor body (11) of the sensor strip (10) when the insertion protrusion (40) is inserted into an insertion hole (12). A corner of one end of the sensor body (11) of the sensor strip (10) may be recessed inwardly. When the insertion protrusion (40) is inserted into the insertion hole (12), the recessed corner comes into close contact with the fixing protrusion (50), and as a result, the sensor strip (10) is firmly fixed to the fixing plate (20).

Hereinafter, a method of analyzing a sample using the biosensor according to the present invention will be described (referring to Figs. 1 to 4).

First, with the biosensor according to the present invention, a detection sample containing the detection substance which specifically reacts with a target analyte in a sample solution is injected into the reaction chambers (13) through the sample injection holes (30). At that time, the detection structures (15) arranged across the internal space of the reaction chamber (13) are immersed in the detection sample and the detection substance is immobilized on the thin film layer (19) of the detection structure (15). After the immobilization of the detection substance on the detection structure (15), the biosensor is arranged in a spectroscopic analyzer and then absorbance is measured while irradiating a light toward the opposite surface of the fixing plate (20). However, the aforementioned absorbance measurement does not necessarily have to be performed.

As described earlier, when the detection substance is immobilized on the detection structure (15), a sample solution is injected into the internal space of the reaction chamber (13) through the sample injection hole (30) such that the detection structure (15) is immersed in the sample solution. At that time, the detection substance in the detection structure (15) reacts with the target analyte in the sample solution. For example, antibody-antigen reaction is induced when the detection substance is an antibody and the target analyte is an antigen.

A sample can be analyzed by arranging the biosensor in a spectroscopic analyzer while immersing its detection structure (15) in the sample solution accommodated in the reaction chamber (13). At this time, it is preferable to pre-heat the spectroscopic analyzer before the biosensor is arranged, and to arrange the biosensor in the spectroscopic analyzer as soon as the detection structure (15) is immersed in the sample solution. However, it is not necessary to pre-heat the spectroscopic analyzer in advance.

Although the present invention has been described herein with reference to the specific embodiments, these embodiments do not serve to limit the invention and are set forth for illustrative purposes. It will be apparent to those skilled in the art that modifications and improvements can be made without departing from the spirit and scope of the invention.

Such simple modifications and improvements of the various embodiments disclosed herein are within the scope of the present invention, and the specific scope of the present invention will be additionally defined by the appended claims.

### INDUSTRIAL APPLICABILITY

The biosensor according to the present invention permits to quantitatively detect a sample by generating an LSPR phenomenon, and to easily induce the reaction between biological samples or between biological and non-biological samples without a separate sample pretreatment process. Therefore, an industrial applicability of the biosensor is recognized.

### REFERENCE NUMERALS

- 10: sensor strip
- 11: sensor body
- 12: insertion hole
- 13: reaction chamber
- 15, 15a, 15b, 15c: detection structure
- 16: substrate
- 19: thin film layer
- 20: fixing plate
- 21: light passing hole
- 30: insertion hole
- 40: insertion protrusion
- 50: fixing protrusion

## Claims

1. A biosensor comprising at least one sensor strip including a sensor body with a predetermined length, a plurality of reaction chambers recessed from one surface of the sensor body, and one or more detection structures arranged in each of the reaction chambers.

2. The biosensor according to claim 1, further comprising a fixing plate having a surface to which the sensor strip is detachably attached.

3. The biosensor according to claim 1, wherein the detection structure comprises a substrate arranged across the internal space of the reaction chamber; and a thin film layer that is formed on at least one of the both surfaces of the substrate by dispersedly disposing conductive nanoparticles or nanostructures that cause the LSPR phenomenon and reacts with a target analyte in a sample.

4. The biosensor according to claim 1, wherein the detection structure is provided in plurality and the detection structures are horizontally spaced apart from each other in the reaction chamber.

5. The biosensor according to claim 1, further comprising sample injection holes recessed from one surface of the sensor body so as to be in communication with the reaction chambers.

6. The biosensor according to claim 2, wherein the fixing plate comprises light passing holes perforated along its thickness direction so that the light irradiated from the direction of the surface of the fixing plate enters the detection structure or the light irradiated to the direction of the surface of the fixing plate passes through it.

7. The biosensor according to claim 6, wherein the light passing hole is formed in plurality to correspond one-to-one to a set of the detection structures arranged in each reaction chamber.

8. The biosensor according to claim 2, further comprising an insertion protrusion protruding from one surface of the fixing plate wherein the sensor body is recessed or perforated to form an insertion recess into which the insertion protrusion is inserted such that the sensor strip is attached to the fixing plate.

9. The biosensor according to claim 8, further comprising a fixing protrusion spaced from the insertion protrusion and protruding from one surface of the fixing plate such that the insertion protrusion comes into contact with an inwardly recessed corner of one end of the sensor body when inserted into the insertion hole.
